(19) Europäisches Patentamt
European Patent Office
Office européen des brevets

(11) EP 1 584 320 A1

(12) EUROPEAN PATENT APPLICATION
published in accordance with Art. 158(3) EPC

(43) Date of publication:
**12.10.2005 Bulletin 2005/41**

(21) Application number: **03777239.9**

(22) Date of filing: **04.12.2003**

(51) Int Cl.[7]: **A61K 7/00**, A61K 7/06, A61K 7/075, A61K 7/08, A61K 7/09, A61K 7/13

(86) International application number:
**PCT/JP2003/015520**

(87) International publication number:
**WO 2004/052318 (24.06.2004 Gazette 2004/26)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **06.12.2002 JP 2002354637**
**16.12.2002 JP 2002363247**
**07.03.2003 JP 2003060816**
**07.03.2003 JP 2003060829**

(71) Applicant: **Seiwa Kasei Company, Limited**
**Osaka (JP)**

(72) Inventors:
- **YOSHIOKA, Masato**
  **c/o Seiwa Kasei Company, Limited**
  **Higashiosaka-shi, Osaka 579-8004 (JP)**
- **ADACHI, Takashi**
  **c/o Seiwa Kasei Company, Limited**
  **Higashiosaka-shi, Osaka 579-8004 (JP)**
- **UEHARA, Keiichi**
  **c/o Seiwa Kasei Company, Limited**
  **Higashiosaka-shi, Osaka 579-8004 (JP)**

(74) Representative: **Vossius & Partner**
**Siebertstrasse 4**
**81675 München (DE)**

# (54) COSMETIC PREPARATION CONTAINING GLYCERYLAMINO ACID DERIVATIVE

(57) Skin care cosmetics, such as lotions, creams and milky lotions, and hair cosmetics, such as hair creams, hair conditioners, hair rinses and shampoos, being excellent in moisturizing effect, applicability, stability and safety and **characterized in** containing an amino acid N-glyceryl derivative produced by reacting neutral, basic or acidic α-amino acid or salt thereof with glycidol or 3-halo-1,2-propanediol.

EP 1 584 320 A1

## Description

FIELD OF THE INVENTION

The present invention relates to cosmetics. In more detail, it relates to cosmetics containing amino acid N-glyceryl derivatives which have excellent moisturizing effect, safety and ability to improve skin damage, and also favorable feel in application, wherein such cosmetics are employed for skin care cosmetics being excellent in skin affinity, and not sticky to skin, and giving moist feel to skin, or for hair cosmetics which exerting gloss, moist feel, improving combability and smoothness.

DESCRIPTION OF THE RELATED ART

Moisturizers are conventionally applied as a major component to cosmetics such as skin care cosmetics or hair cosmetics; for example, 1,3-butylene glycol, propylene glycol, polyethylene glycol, glycerin, amino acids and polypeptides are applied as a moisturizer by the reasons of their good availability, safety and moisturizing effect.

Some of these moisturizers, however, have disadvantages for skin in terms of practical use such as lack of moisturizing effect to impart moist feel and problems of stickiness to skin, or for hair in terms of unsatisfied function in gloss, moist feel, smoothness and improving combability. Besides these, some kinds of moisturizers have other problems concerning oxidation stability and odor.

The present invention intends to solve problems of conventional arts such as described above, and to provide cosmetics having excellent moisturizing effect, applicability, stability and safety, wherein such cosmetics include skin care cosmetics having excellent skin affinity, less stickiness and imparting moist feel to skin, or hair cosmetics exerting gloss, moist feel, improving combability and smoothness.

SUMMARY OF THE INVETION

The inventors of the invention have extensively studied to solve problems described above, and found that cosmetics containing amino acid N-glyceryl derivative represented by the following formula (I) or salt thereof;

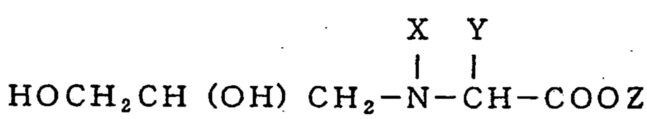

(I)

, wherein X represents hydrogen atom, - $CH_2CH(OH)CH_2OH$ group or alkyl group having 1 to 4 carbon atoms, Y represents a side chain of α-amino acid, and Z represent hydrogen atom, alkali metal, ammonium, organic ammonium or - $CH_2CH(OH)CH_2OH$ group, have excellent moisturizing effect, applicability, stability and safety, impart advantageous effects on skin care cosmetics, such as excellent skin affinity, less stickiness to skin and moist skin feel, and impart advantageous effects on hair cosmetics, such as gloss, moist feel, improving combability and smoothness; and have accomplished the present invention.

The present invention provides the cosmetics containing amino acid N-glyceryl derivative represented by the formula (I) or salt thereof.

The group of Y represented in the formula (I) is a group which can be a side chain of α-amino acid. This α-amino acid is represented by the following formula (II);

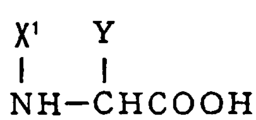

(II)

, wherein $X^1$ represents hydrogen atom or alkyl group having 1 to 4 carbon atoms, and Y represents same meaning described above. Examples of the α- amino acid include neutral amino acids (which also involve amino acids containing sulfur, such as cystine, methionine and the like), acidic amino acids in which the Y has -COOH group, and basic amino acids in which the Y has -$NH_2$ group, guanidino group or imidazole group.

The ammonium represented by Z in the formula (I) is a group represented by -$NH_4^+$. The organic ammonium

is a group represented by $-NR_4^+$, wherein R represents hydrogen atom, alkyl groups such as methyl, ethyl and the like, or alkyl groups in which any of hydrogen atom is replaced by another group, such as hydroxymethyl group, hydroxyethyl group, 2-methyl-1,3-propanediol-2-yl group, 2-methyl-1-propanolamine-2-ylgroup, and the like, and at least one of Rs is not hydrogen atom.

Examples if the salt of amino acid N-glyceryl derivative include acid addition salts derived from the nitrogen atom in the formula (I); further includes base addition salts derived from the - COOH group when Y contains - COOH group, or acid addition salts derived from the nitrogen atom of the $-NH_2$ group, guanidino group or imidazole group when Y contains $-NH_2$ group, guanidino group or imidazole group.

Examples of the acid addition salt includes organic salts such as acetate, lactate, glycolate, citrate, malate, tartarate, succinate, adipate and the like, and inorganic salts such as hydrochloride, sulfate, phosphate and the like. Examples of the base addition salts include alkali metal salts such as sodium salt, potassium salt and the like; alkali salts such as ammonium salt, triethanolamine salt, monoethanolamine salt, 2-amino-2-methyl-1,3-propanediol salt, 2-amino-2-methyl-1-propanolamine salt and the like.

The cosmetics of the invention are exemplified by skin care cosmetics and hair cosmetics.

DETAILED DESCRIPTION OF THE INVENTION

The present invention will be explained in more detail below.

The amino acid N-glyceryl derivative represented by the formula (I) or salt thereof which is contained in the cosmetics of the present invention, can be obtained by reacting, for example, α-amino acid represented by the formula (II) or salt thereof with glycidol or 3-halo-1,2-propanediol.

After an amino acid N-glyceryl derivative represented by the formula (I) was obtained by above described method, it can be transformed to acid addition salts or base addition salt respectively by acid agents or alkali agents. For example, when an amino acid N-glyceryl derivative represented by the formula (I) exhibits acidity or alkalinity depending a type thereof, it may be transformed to a salt by acid agents or alkali agents according to application objected.

Examples of the α-amino acid represented by the formula (II) which is the starting material for the compound represented by the formula (I) include neutral amino acids such as glycine, alanine, valine, leucine, isoleucine, serine, threonine, phenylalanine, tyrosine, tryptophan, sarcosine, N-methylalanine, a - aminobutyric acid, cystine, methionine, cysteine, proline, hydroxyproline and the like; basic amino acids such as lysine, hydroxylysine, arginine, histidine, ornithine and the like; and acidic amino acids such as aspartic acid, glutamic acid and the like.

Examples of another starting materials which produce the compound represented by the formula (I) by reacting with the a-amino acid represented by the formula (II) include glycidol (i.e. 2,3-epoxy-1-propanol), 3-halo-1,2-propanediol such as 3-chloro-1,2-propanediol and 3-bromo-1,2-propanediol.

The reaction of α-amino acid represented by the formula (II) or salt thereof with glycidol or 3-halo-1, 2-propanediol in order to produce amino acid N-glyceryl derivative represented by the formula (I) or salt thereof, can be carried out in water solvent or organic solvents containing water, under the presence of alkali. Specifically, the α- amino acid represented by the formula (II) is dissolved in water solvent or an organic solvent containing water and pH of this solution is adjusted, followed by being subjected to reaction, while being heated and agitated, by dropwise addition of glycidol or 3-halo-1, 2-propanediol, and then, after the reaction being completed, pH of the reactant is adjusted with an acidic agent to obtain an amino acid N-glyceryl derivative.

Examples of the solvents used for the reaction include water, lower alcohols containing water, acetone containing water, dimethylformamide containing water, dimethylsulfoxide containing water, methyl cellosolve containing water and N-methylpyrrolidone containing water, and mixture thereof. Content of water is preferably 20 % or more.

The pH of reaction solution is preferably 8 to 11 when α-amino acid represented by the formula (II) or salt thereof is subjected to reaction with glycidol or 3-halo-1,2-propanediol in order to produce the amino acid N-glyceryl derivative represented by the formula (I) or salt thereof. If pH is 8 or lower, reaction rate is unfavorably decreased; if pH is 11 or more, although the reaction rate is increased, odor generation or coloration occurs and results in undesirable result.

Depending on the kind of α-amino acid represented by the formula (II), alkali agents or acid agents may be appropriately employed to adjust the pH in a range of 8 to 11. Examples of the alkali agents employed include potassium hydroxide, sodium hydroxide, lithium hydroxide, sodium carbonate and potassium carbonate. Examples of the acid agents employed include organic acids such as acetic acid, lactic acid, glycolic acid, citric acid, malic acid, tartaric acid, succinic acid and adipic acid; and inorganic acids such as hydrochloric acid, sulfuric acid and phosphoric acid.

Reaction temperature is preferably 30 to 90°C . If the reaction is carried out at 30°C or lower, reaction rate is unfavorably decreased; if reaction temperature is 90°C or higher, odor generation and coloration tend to easily occur.

Period of time to drop glycidol or 3-halo-1,2-propanediol for reaction depends on the amount to be reacted; after the dropping being finished, the reaction solution is preferably left in agitation for 3 to 15 hours under being heated

to complete the reaction.

After reaction being completed, the reactant is subjected to pH adjustment with optional acid agents to obtain an amino acid N-glyceryl derivative represented by the formula (I) or a salt thereof which is to be contained in cosmetics of the present invention. The final pH of the reactant is preferably 4 to 9 to be contained as a moisturizer in the cosmetics of the present invention.

Examples of the acids applied for pH adjustment include organic acids such as acetic acid, lactic acid, glycolic acid, citric acid, malic acid, tartaric acid, succinic acid and adipic acid; and inorganic acids such as hydrochloric acid, sulfuric acid and phosphoric acid.

After this pH adjustment being finished, the reactant is contained in the cosmetics of the present invention, as itself in a form of liquid or by concentration when the reactant is the solution of water solvent, or after concentration under reduced-pressure or pulverization when the reaction solution contains an organic solvent.

Specific examples of thus produced amino acid N-glyceryl derivative represented by the formula (I) include followings.

When the α-amino acid represented by the formula (II) applied as the raw material is neutral amino acids, included are N-glycerylglycine (i.e. N-2,3-dihydroxypropylglycine), N-glycerylalanine, N-glycerylvaline, N-glycerylleucine, N-glycerylisoleucine, N-glycerylserine, N-glycerylthreonine, N-glycerylcysteine, N-glycerylcystine, N-glycerylmethionine, N-glycerylphenylalanine, N-glyceryltyrosine, N,N'-diglycerylcystine, N,N-diglycerylglycine, N,N-diglycerylalanine, N,N-diglycerylvaline, N,N-diglycerylleucine, N,N-diglycerylisoleucine, N,N-diglycerylserine, N,N-diglycerylthreonine, N,N-diglycerylcystine, N,N-diglycerylmethionine, N,N-diglycerylphenylalanine, N,N-diglyceryltyrosine, N-glyceryltryptophan (in which α-amino group being added with glyceryl group), N,N-diglyceryltryptophan (in which α-amino group being added with glyceryl group), N-glycerylsarcosine (N-2,3-dihydroxypropylsarcosine).

When the amino acid represented by the formula (II) is basic amino acids, included are, N-glyceryllysine (i.e. $N^2$-2,3-dihydroxypropyllysine or $N^6$-2,3-dihydroxypropyllysine), N-glycerylhydroxylysine (i.e. $N^2$-2,3-dihydroxypropylhydroxylysine or $N^6$-2,3-dihydroxypropylhydroxylysine), N-glycerylarginine (in which α - amino group being added with glyceryl group), N-glycerylhistidine (in which α-amino group being added with glyceryl group), N-glycerylornithine (in which α-amino group being added with glyceryl group), $N^2$,$N^6$-diglyceryllysine, $N^2$,$N^6$-diglycerylhydroxylysine, N, N-diglycerylarginine (in which α- amino group being added with two glyceryl groups), N, N-diglycerylhistidine (in which α - amino group being added with two glyceryl groups), $N^2$,$N^6$-diglycerylornithine.

When the amino acid represented by the formula (II) is acidic amino acids, included are N-glycerylaspartic acid (i.e. N-2,3-dihydroxypropylaspartic acid), N-glycerylglutamic acid, N,N-diglycerylaspartic acid, N,N-diglycerylglutamic acid.

The glyceryl group described above means 2,3-dihydroxypropyl group.

Of these amino acids, the product produced by using basic amino acids as the amino acid represented by the formula (II), is preferable due to its excellent substantivity to hair and ability to improve skin damage.

The product produced by the reaction of α-amino acid represented by the formula (II) or the salt thereof with glycidol or 3-halo-1,2-propanediol, is usually composed of the aforementioned compounds as a major component; besides such major component, such product often contains compounds represented by the following formula (III) and formula (IV):

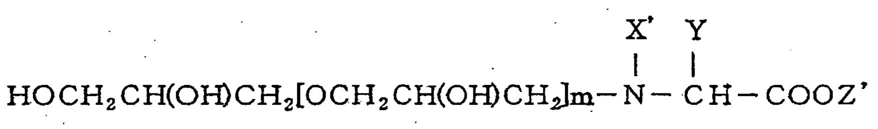

(III)

, wherein X' represents hydrogen atom, alkyl group having 1 to 4 carbon atoms or $-[CH_2CH(OH)CH_2O]nCH_2CH(OH_2CH$ group, n is an integer of 0, 1 or more, Y represents a side chain of α-amino acid, Z'representshydrogenatom, alkalimetals, ammonium, organic ammonium group or $-[CH_2CH(OH)CH_2O]lCH_2CH(OH)CH_2OH$ group, l is an integer of 0, 1 or more, and m is an integer of 1 or more; and

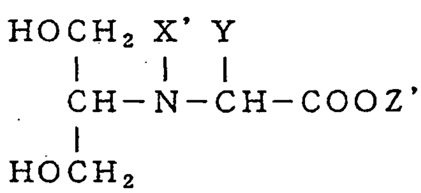

(IV)

, wherein X', Y and Z' represent same meaning described above. The cosmetics of the invention may contain these compounds together with the amino acidN-glyceryl derivative represented by the formula (I) or salt thereof.

Since the amino acid N-glyceryl derivative represented by the formula (I) or salt thereof have excellent moisturizing effect, by blending such amino acid N-glyceryl derivative or salt thereof to skin care cosmetics or hair cosmetics, the skin care cosmetics or hair cosmetics having excellent moisturizing effect can be obtained. Furthermore, since the amino acid N-glyceryl derivative represented by the formula (I) or salt thereof, has excellent applicability, stability, safety and ability to improve skin damage; when being used to skin care cosmetics, such agents exert superior effects of moisturizing effect together with applicability (not being sticky but giving moist feel), stability, safety and ability to improve skin damage; and when being applied to hair cosmetics, such cosmetics impart superior effects of moisturizing effect together with applicability (not being sticky but giving moist feel) and gloss.

Two or more types of amino acid N-glyceryl derivative represented by the formula (I) or salts thereof may be blended in the cosmetics of the present invention. When two or more types of amino acid N-glyceryl derivative being blended, the two or more types of amino acid N-glyceryl derivatives may be independently produced, and then blended in combination, or a mixture of some types of amino acid N-glyceryl derivative may be pre-produced, and then directly blended to skin care cosmetics or hair cosmetics.

Typical examples of the cosmetics of the present invention include cosmetics, drugs and quasi-drugs which are applied to skin (including scalp) and hair; specifically included are , for example, skin care cosmetics such as lotions, milky lotions, creames and face masks; make-up cosmetics such as foundations, lip sticks and eye shadows, and body cosmetics; and hair cosmetics such as shampoos, hair rinses, hair conditioners, hair creams, hair dyes, hair colors, pre- or post-treatment agents for hair dyeing and coating agents for split hair.

The content (amount blended in cosmetics) of the amino acid N-glyceryl derivative represented by the formula (I) or salt thereof to the cosmetics of the present invention, varies depending on the type of cosmetics applied, and is preferably 0.05 to 20 % by weight, more preferably 0.1 to 10 % by weight. If the content is such range or less, effects to improve moisturizing effect, feel in application and the like may not be fully exerted; if the content being such range or more, troubles on formulation or stickiness to skin or hair may caused.

The skin care cosmetics and hair cosmetics of the present invention may optionally blend, besides the amino acid N-glyceryl derivative represented by the formula (I) or salt thereof of the present invention, for example, oily substances, moisturizers; surfactants, thickeners, high molecular substances, powders, drugs, antioxidants, ultraviolet absorbers, perfumes, chelating agents, extracts of animal or plant, and hydrolyzed peptides or derivatives thereof produced by hydrolyzing protein derived from animal or plant.

Formulation type of the cosmetics of the present invention is optional, any types such as solution system, soluble system, emulsion system, gel system, powder dispersing system or water-oil two phase system, are possible; and can be prepared according to objected products by blending any of optional component mentioned above with the amino acid N-glyceryl derivative represented by the formula (I) or salt thereof.

The cosmetics containing the amino acid N-glyceryl derivative represented by the formula (I) or salt thereof of the present invention, are excellent in moisturizing effect, applicability, stability, safety and ability to improve skin damage. When being applied for skin care cosmetics, such agents have remarkable affinity to skin, and are not sticky to skin but impart moist feel to skin; and, when being applied for hair cosmetics, such cosmetics are not sticky, impart favorable moisturizing effect, moist feel and gloss, improve combability and allow hair smooth.

The present invention is explained in more detail by referring Examples. The present invention should not be construed to be limited to the examples. The amount of each component blended in the following Examples and Comparative Examples, is represented by part by weight; if the amount does not represent solid content, the solid concentration is exhibited in a parenthesis following to the component name. In Examples and Comparative examples, term of "blended" is used in place of "contained" for each component.

In advance explaining Examples, the examples synthesizing the amino acid N-glyceryl derivative of the present invention which is applied in Examples, are mentioned.

Followings are methods to measure contents of total nitrogen and amino nitrogen in Synthesis Examples.

[Measurement of Total Nitrogen Content]

The content of total nitrogen of an amino acid or a reaction product was measured by a nitrogen-carbon analyzer, SUMIGRAPH NC-95A (trade name) manufactured by SUMITOMO CHEMICAL, Co., Ltd. in which an organic sample is oxidized in an electric furnace to convert nitrogen to $NO_2$ gas and carbon to $CO_2$ gas, followed by each gas being independently quantified by a gas chromatography. Ureawas employed as a standard substance for quantitative analysis.

[Measurement of Amino Nitrogen Content]

The content of amino nitrogen of an amino acid or a reaction product was measured by a amino nitrogen analyzer, SUMIGRAPH N-350 (trade name) manufactured by SUMITOMO CHEMICAL, in which the Van Slyke's method is applied, and nitrogen atom of an amino acid is automatically reacted with nitrous acid and then the nitrogen gas ($N_2$) generated is quantified by a gas chromatography. Methionine was employed as a standard substance for quantitative analysis.

Synthesis Example 1 : Synthesis of a glyceryl derivative of glycine

In 3 liter three-neck round-bottom flask, 75.1 g of glycine and 675 g of water were put in and the pH of the solution was adjusted to about 9.5 by adding aqueous sodium hydroxide, followed by, while being heated at about 60°C and agitated, being gradually dropped with 59.3 g of glycidol over 1 hour. After the dropping finished, the solution was still left under agitation for 6 hours to complete reaction. The reaction solution was cooled down to a room temperature and then its pH was adjusted to about 6.5 by adding dilute hydrochloric acid, followed by concentration under a reduced pressure to obtain a viscous substance. The viscous substance was added with 1344 g of methanol and agitated to obtain a powder-form precipitate, followed by the precipitate being filtered. It was found that the precipitate was mainly composed of unreacted glycine from the result of analyses by a thin-layer chromatography (hereunder abbreviated as TLC) and a gas chromatography (hereunder abbreviated as GC). The methanol solution having eliminated the powder-form precipitate, was subjected to distilling solvent off under a reduced pressure to obtain 101.5 g of viscous substance which was a glyceryl derivative of glycine. Contents of total nitrogen, total carbon and amino nitrogen were measured on the glyceryl derivative of glycine obtained and glycine employed as raw material. The results were as follows.

| Glyceryl derivative of glycine | |
|---|---|
| Total nitrogen content | 10.68 % |
| Total carbon content | 36.68 % |
| Amino nitrogen content | 3.54 % |

| Glycine (The enclosed by parenthesis is a calculated value) | |
|---|---|
| Total nitrogen content | 18.29 % (18.66 %) |
| Total carbon content | 31.36 % (32.00 %) |
| Amino nitrogen content | 18.47 % (18.66 %) |

From the results of analysis by a thin-layer chromatography (hereunder abbreviated as TLC) and a gas chromatography (hereunder abbreviated as GC), it was found that the precipitate described above was mainly composed of unreacted glycine.

In the analysis result carried out by TLC under the conditions described below on the glyceryl derivative of glycine, a spot was detected at the site of 0.28 of Rf value and there were no other spots except one or two of faint spots; according to this Rf value and the results of total nitrogen content, total carbon content and amino nitrogen content, it was understood the major component was N-glycerylglycine.

| Analysis conditions of TLC | |
|---|---|
| Thin-layer plate | Silica gel manufactured by Merck Ltd. 60 (trade name) |
| Eluent | Phenol : water = 4 :1 (W/W) |
| Detection method | Ultraviolet (365 nm) radiation |

A portion of the glyceryl derivative of glycine was subjected to trimethylsilylation by applying N,O-Bis(trimethylsilyl)acetamide (BSA), followed by being subjected to GC analysis under the conditions described below; a peak of major component was observed in the retention time of 10.7 minutes, besides two to three of extremely small peaks derived from other components.

| Analysis conditions of GC | |
|---|---|
| column packing material | 5 % SE-30, 50 cm |
| Carrier gas | Nitrogen 30 ml/minute |
| Detector | FID (Hydrogen flame detector) |
| Inlet temperature | 310°C |
| Detector temperature | 310°C |
| Temperature condition | Heating from 80°C up to 300°C by a rate of 8°C/ minute |

Synthesis Example 2 : Synthesis of a glyceryl derivative of alanine

In 3 liter three-neck round-bottom flask, 133.6 g of alanine and 1200 g of water were put in and the pH of the solution was adjusted to about 9.5 by adding aqueous sodium hydroxide, followed by, while being heated at about 60°C and agitated, being gradually dropped with 111.2 g of glycidol over 1 hour. After the dropping finished, the solution was still left under agitation for 5 hours to complete reaction. The reaction solution was cooled down to a room temperature and then its pH was adjusted to about 6.5 by adding dilute hydrochloric acid, followed by concentration under a reduced pressure to obtain a viscous substance. The viscous substance was added with 600 g of methanol, and agitated, and then insoluble matters were removed by filtration. The filtrate was subjected to distilling solvent off under a reduced pressure to obtain 208 g of viscous liquid substance. Contents of total nitrogen, total carbon and amino nitrogen were measured on the glyceryl derivative of alanine obtained and alanine employed as raw material. The results were as follows.

| Glyceryl derivative of alanine | |
|---|---|
| Total nitrogen content | 9.52 % |
| Total carbon content | 41.25 % |
| Amino nitrogen content | 2.99 % |

| Alanine (The enclosed by parenthesis is a calculated value) | |
|---|---|
| Total nitrogen content | 15.57 % (15.73 %) |
| Total carbon content | 39.63 % (40.44 %) |
| Amino nitrogen content | 15.42 % (15.73 %) |

TLC analysis and GC analysis of the obtained substance were carried out according to the same conditions as in Synthesis Example 1, a main spot was detected at the site of 0.36 of Rf value in TLC and a main peak was observed in the retention time of 9.1 minutes in GC analysis, besides two to three of extremely small peaks derived from other components. According to this Rf value and the above results of total nitrogen content, total carbon content and amino nitrogen content, it was understood the major component was N-glycerylalanine.

Synthesis Example 3 : Synthesis of a glyceryl derivative of sarcosine

In 3 liter three-neck round-bottom flask, 89.1 g of sarcosine and 801 g of water were put in and the pH of the solution was adjusted to about 9.5 by adding aqueous sodium hydroxide, followed by, while being heated at about 60°C and agitated, being gradually dropped with 74.1 g of glycidol over 1 hour. After the dropping finished, the solution was still left under agitation for 7 hours to complete reaction. The reaction solution was cooled down to a room temperature and then its pH was adjusted to about 6.5 by adding dilute hydrochloric acid, followed by concentration under a reduced pressure to obtain a viscous substance. The viscous substance was added with 1630 g of ethanol, and agitated, and then insoluble matters were removed by filtration. The filtrate was subjected to distilling solvent off under a reduced pressure to obtain 124 g of viscous liquid substance. Contents of total nitrogen, total carbon and amino nitrogen were measured on the glyceryl derivative of sarcosine obtained and sarcosine employed as raw material. The results were as follows.

| Glyceryl derivative of sarcosine | |
|---|---|
| Total nitrogen content | 10.19 % |
| Total carbon content | 40.89 % |
| Amino nitrogen content | 4.48 % |

| Sarcosine (The enclosed by parenthesis is a calculated value) | |
|---|---|
| Total nitrogen content | 15.42 % (15.73 %) |
| Total carbon content | 39.23 % (40.44 %) |
| Amino nitrogen content | 15.26 % (15.73 %) |

TLC analysis and GC analysis of the obtained substance were carried out according to the same conditions as in Synthesis Example 1, a main spot was detected at the site of 0.51 of Rf value in TLC and a main peak was observed in the retention time of 8.9 minutes in GC analysis, besides two to three of extremely small peaks derived from other components. According to this Rf value and the above results of total nitrogen content, total carbon content and amino nitrogen content, it was understood the major component was N-glycerylsarcosine.

Synthesis Example 4 : Synthesis of a glyceryl derivative of phenylalanine

In 3 liter three-neck round-bottom flask, 82.6 g of phenylalanine and 1570 g of water were put in and the pH of the solution was adjusted to about 9.5 by adding aqueous sodium hydroxide, followed by, while being heated at about 60°C and agitated, being gradually dropped with 37.1 g of glycidol over 1 hour. After the dropping finished, the solution was still left under agitation for 8 hours to complete reaction. The reaction solution was cooled down to a room temperature and then its pH was adjusted to about 6.5 by adding dilute hydrochloric acid, followed by concentration under a reduced pressure to obtain a wax-like substance. The wax-like substance was added with 1300 g of methanol, and agitated to precipitate insoluble matters. The insoluble matters were removed by filtration. Then, it was dried to obtain 78 g white powder. Contents of total nitrogen, total carbon and amino nitrogen were measured on the glyceryl derivative of phenylalanine obtained and phenylalanine employed as raw material. The results were as follows.

| Glyceryl derivative of phenylalanine | |
|---|---|
| Total nitrogen content | 6.44 % |
| Total carbon content | 58.95 % |
| Amino nitrogen content | 2.82 % |

| Phenylalanine (The enclosed by parenthesis is a calculated value) | |
|---|---|
| Total nitrogen content | 8.23 % (8.48 %) |
| Total carbon content | 64.12 % (65.43 %) |
| Amino nitrogen content | 8.40 % (8.48 %) |

TLC analysis and GC analysis of the white powder were carried out according to the same conditions as in Synthesis Example 1, a main spot was detected at the site of 0.34 of Rf value in TLC and a main peak was observed in the retention time of 14.9 minutes in GC analysis , besides two to three of extremely small peaks derived from other components. According to this Rf value and the above results of total nitrogen content, total carbon content and amino nitrogen content, it was understood the major component was N-glycerylphenylalanine.

Synthesis Example 5 : Synthesis of a glyceryl derivative of leucine

In 3 liter three-neck round-bottom flask, 26.2 g of leucine and 1050 g of water were put in and the pH of the solution was adjusted to about 9.5 by adding aqueous sodium hydroxide, followed by, while being heated at about 60°C and agitated, being gradually dropped with 14.8 g of glycidol over 1 hour. After the dropping finished, the solution was still left under agitation for 8 hours to complete reaction. The reaction solution was cooled down to a room temperature and then its pH was adjusted to about 6.5 by adding dilute hydrochloric acid, followed by concentration under

a reduced pressure to obtain a wax-like substance. The wax-like substance was added with 610 g of methanol, and agitated to precipitate insoluble matters. The insoluble matters were removed by filtration. Then, it was dried to obtain 26.6 g white powder. Contents of total nitrogen, total carbon and amino nitrogen were measured on the glyceryl derivative of leucine obtained and leucine employed as raw material. The results were as follows.

| Glyceryl derivative of leucine | |
|---|---|
| Total nitrogen content | 7.58 % |
| Total carbon content | 50.67 % |
| Amino nitrogen content | 3.04 % |

| Leucine (The enclosed by parenthesis is a calculated value) | |
|---|---|
| Total nitrogen content | 10.53 % (10.68 %) |
| Total carbon content | 53.84 % (54.94 %) |
| Amino nitrogen content | 10.36 % (10.68 %) |

TLC analysis and GC analysis of the white powder were carried out according to the same conditions as in Synthesis Example 1, a main spot was detected at the site of 0.61 of Rf value in TLC and a main peak was observed in the retention time of 11.1 minutes in GC analysis , besides two to three of extremely small peaks derived from other components. According to this Rf value and the above results of total nitrogen content, total carbon content and amino nitrogen content, it was understood the major component was N-glycerylleucine.

Synthesis Example 6 : <u>Synthesis of a glyceryl derivative of proline</u>

In 3 liter three-neck round-bottom flask, 111.5 g of proline and 1035 g of water were put in and the pH of the solution was adjusted to about 9.5 by adding aqueous sodium hydroxide, followed by, while being heated at about 60°C and agitated, being gradually dropped with 74.1 g of glycidol over 1 hour. After the dropping finished, the solution was still left under agitation for 5 hours to complete reaction. The reaction solution was cooled down to a room temperature and then its pH was adjusted to about 6.5 by adding dilute hydrochloric acid, followed by concentration under a reduced pressure to obtain a viscous substance. The viscous substance was added with 300 g of methanol, and agitated, and then yielded insoluble matters were removed by filtration. The filtrate was subjected to distilling solvent off under a reduced pressure to obtain 136 g of viscous liquid.

TLC analysis and GC analysis of the viscous liquid were carried out according to the same conditions as in Synthesis Example 1, a main spot was detected at the site of 0.59 of Rf value in TLC and a main peak was observed in the retention time of 11.6 minutes in GC analysis, besides two to three of extremely small peaks derived from other components. According to the results, it was presumed the major component was N-glycerylproline.

Synthesis Example 7 : <u>Synthesis of a glyceryl derivative of serine</u>

In 3 liter three-neck round-bottom flask, 105.1 g of serine and 950 g of water were put in and the pH of the solution was adjusted to about 9.5 by adding aqueous sodium hydroxide, followed by, while being heated at about 60°C and agitated, being gradually dropped with 74 g of glycidol over 1 hour. After the dropping finished, the solution was still left under agitation for 5 hours to complete reaction. The reaction solution was cooled down to a room temperature and then its pH was adjusted to about 6.5 by adding dilute hydrochloric acid, followed by concentration under a reduced pressure. The obtained substance was added with 1500 g of methanol, and heated and agitated. The yielded insoluble matters were removed by filtration to obtain 125 g white powder. Contents of total nitrogen, total carbon and amino nitrogen were measured on the glyceryl derivative of serine obtained and serine employed as raw material. The results were as follows.

| Glyceryl derivative of serine | |
|---|---|
| Total nitrogen content | 8.74 % |
| Total carbon content | 36.75 % |
| Amino nitrogen content | 3.16 % |

| Serine (The enclosed by parenthesis is a calculated value) | |
|---|---|
| Total nitrogen content | 13.22 % (13.33 %) |
| Total carbon content | 33.68 % (34.02 %) |
| Amino nitrogen content | 13.06 % (13.33 %) |

TLC analysis and GC analysis of the white powder were carried out according to the same conditions as in Synthesis Example 1, a main spot was detected at the site of 0.23 of Rf value in TLC and a main peak was observed in the retention time of 12.8 minutes in GC analysis, besides two to three of extremely small peaks derived from other components. According to this Rf value and the above results of total nitrogen content, total carbon content and amino nitrogen content, it was understood the major component was N-glycerylserine.

Synthesis Example 8 : Synthesis of a glyceryl derivative of lysine

To 3 liter three-neck round-bottom flask put with 1644 ml of water, 182.7 g of lysine hydrochloride was added and dissolved, followed by adjusting the pH of the solution to about 9.5 by adding 20 % aqueous sodium hydroxide. While being heated at about 60°C and agitated, the aqueous solution was gradually dropped with 74 g of glycidol over 30 minutes. After the dropping finished, the solution was still left at 60°C under agitation for 5 hours to complete reaction. After completion of the reaction, the reaction solution was cooled down to a room temperature and then its pH was adjusted to 6.8 by adding hydrochloric acid, followed by concentration under a reduced pressure to obtain 258g of glyceryl derivative of lysine.

Contents of total nitrogen, total carbon and amino nitrogen were measured on the glyceryl derivative of lysine obtained and lysine hydrochloride employed as raw material. The results were as follows.

| Glyceryl derivative of lysine | |
|---|---|
| Total nitrogen content | 8.25 % |
| Total carbon content | 33.74 % |
| Amino nitrogen content | 5.63 % |

| Lysine hydrochloride(The enclosed by parenthesis is a calculated value) | |
|---|---|
| Total nitrogen content | 14.71 % (15.33 %) |
| Total carbon content | 39.02 % (39.41 %) |
| Amino nitrogen content | 14.60 % (15.33 %) |

According to the above results, it was confirmed that 40 % or more of the amino groups in the lysine were reacted with glyceryl group, and it was understood that the major component of the obtained glyceryl derivative of lysine was N-glyceryllysine hydrochloride.

Synthesis Example 9 : Synthesis of a glyceryl derivative of arginine - 1

To 3 liter three-neck round-bottom flask put with 1568 ml of water, 174 g of arginine was added and dissolved, followed by adjusting the pH of the solution to 9.4 by adding 1 N hydrochloric acid. While being heated at about 65°C and agitated, the aqueous solution was gradually dropped with 74 g of glycidol over 1 hour. After the dropping finished, the solution was still left at 65°C under agitation for 9 hours to complete reaction. After completion of the reaction, the reaction solution was cooled down to a room temperature and then its pH was adjusted to 6.4 by adding 1 N hydrochloric acid, followed by concentration under a reduced pressure to obtain 282g of glyceryl derivative of arginine.

Contents of total nitrogen, total carbon and amino nitrogen were measured on the glyceryl derivative of arginine obtained and arginine employed as raw material. The results were as follows.

| Glyceryl derivative of arginine | |
|---|---|
| Total nitrogen content | 17.71 % |
| Total carbon content | 35.60 % |
| Amino nitrogen content | 1.25 % |

| Arginine (The enclosed by parenthesis is a calculated value) | |
|---|---|
| Total nitrogen content | 31.24 % (32.15 %) |
| Total carbon content | 40.95 % (41.33 %) |
| Amino nitrogen content | 7.78 % (8.04 %) |

According to the above results, it was confirmed that 80 % ormore of the amino groups in the argininewere reacted with glyceryl group, and it was understood that the maj or component of the obtained glyceryl derivative of arginine was N-glycerylarginine hydrochloride.

Synthesis Example 10 : Synthesis of a glyceryl derivative of histidine

To 3 liter three-neck round-bottom flask put with 1886 ml of water, 209 g of histidine hydrochloride 1 hydrate was added and dissolved, followed by adjusting the pH of the solution to 9.3 by adding 20% aqueous sodium hydroxide. While being heated at about 60°C and agitated, the aqueous solution was gradually dropped with 74 g of glycidol over 1 hour. After the dropping finished, the solution was still left at 60°C under agitation for 7 hours to complete reaction. After completion of the reaction, the reaction solution was cooled down to a room temperature and then its pH was adjusted to 6.3 by adding 1 N hydrochloric acid, followed by concentration under a reduced pressure to obtain 259g of crude product.

The obtained resultant was added with 341g of water and dissolved, and then 1263g of ethanol is added and agitated. After the precipitants were removed, the filtrate was distilled under a reduced pressure to obtain 164g of glyceryl derivative of histidine.

Contents of total nitrogen, total carbon and amino nitrogen were measured on the glyceryl derivative of histidine obtained and histidine hydrochloride 1 hydrate employed as raw material. The results were as follows.

| Glyceryl derivative of histidine | |
|---|---|
| Total nitrogen content | 14.39 % |
| Total carbon content | 37.39 % |
| Amino nitrogen content | 1.21 % |

| Histidine hydrochloride 1 hydrate (The enclosed by parenthesis is a calculated value) | |
|---|---|
| Total nitrogen content | 20.22 % (20.04 %) |
| Total carbon content | 36.08 % (34.35 %) |
| Amino nitrogen content | 6.81 % (6.68 %) |

According to the above results, it was confirmed that 70 % or more of the amino groups in the histidine were reacted with glyceryl group, and it was understood that the major component of the obtained glyceryl derivative of histidine was N-glycerylhistidine hydrochloride.

Synthesis Example 11 : Synthesis of a glyceryl derivative of arginine - 2

To 3 liter three-neck round-bottom flask put with 1568 ml of water, 173 g of arginine is added and dissolved, followed by adjusting the pH of the solution to 9.3 by adding aqueous glycolic acid solution. While being heated at about 60°C , the aqueous solution is gradually dropped with 73 g of glycidol over about 1 hour. After the dropping finished, the solution is still left at 60°C under agitation for 6 hours to complete reaction. After completion of the reaction, the reaction solution is cooled down to a room temperature and then its pH is adjusted to 6.4 by adding aqueous glycolic acid solution, followed by concentration under a reduced pressure. Thus, glyceryl derivative of arginine containing N-glycerylarginine glycolate as the major component can be obtained.

Synthesis Example 12 : Synthesis of a glyceryl derivative of tryptophan

To 3 liter three-neck round-bottom flask put with 1830 ml of water, 20.4 g of tryptophan is added and dissolved, followed by adjusting the pH of the solution to 9.2 by adding 20% aqueous sodium hydroxide solution. While being heated at about 60°C, the aqueous solution is gradually dropped with 7.3 g of glycidol over 30 minutes. After the

dropping finished, the solution is still left at 60°C under agitation for 8 hours to complete reaction. After completion of the reaction, the reaction solution is cooled down to a room temperature and then its pH is adjusted to 6.6 by adding hydrochloric acid, followed by concentration under a reduced pressure. Thus, glyceryl derivative of tryptophan containing N-glyceryltryptophan hydrochloride as the major component can be obtained.

Synthesis Example 13 : Synthesis of a glyceryl derivative of aspartic acid

To 3 liter three-neck round-bottom flask put with 1340 ml of water, 173 g of sodium aspartate 1 hydrate was added and dissolved, followed by adjusting the pH of the solution to 9.5 by adding 20 % aqueous sodium hydroxide. While being heated at about 60°C and agitated, the aqueous solution was gradually dropped with 74 g of glycidol over 40 minutes. After the dropping finished, the solution was still left at 60°C under agitation for 8 hours to complete reaction. After completion of the reaction, the reaction solution was cooled down to a room temperature and then its pH was adjusted to 6.5 by adding hydrochloric acid, followed by concentration under a reduced pressure to obtain 286g of glyceryl derivative of aspartic acid. 20% of the product can be considered as sodium chloride yielded by the neutralization.

Contents of total nitrogen, total carbon and amino nitrogen were measured on the glyceryl derivative of aspartic acid and sodium aspartate 1 hydrate employed as raw material. The results were as follows.

| Glyceryl derivative of aspartic acid | |
|---|---|
| Total nitrogen content | 4.85 % |
| Total carbon content | 29.12 % |
| Amino nitrogen content | 1.13 % |

| Sodium aspartate 1 hydrate(The enclosed by parenthesis is a calculated value) | |
|---|---|
| Total nitrogen content | 8.00 % (8.09 %) |
| Total carbon content | 26.81 % (27.75 %) |
| Amino nitrogen content | 7.96 % (8.09 %) |

According to the above results, it was confirmed that 70 % or more of the amino groups in the aspartic acid were reacted with glyceryl group, and it was understood that the major component of the obtained glyceryl derivative of aspartic acid was 1 sodium N-glycerylaspartate.

Synthesis Example 14 : Synthesis of a glyceryl derivative of glutamic acid

To 3 liter three-neck round-bottom flask put with 1060 ml of water, 118 g of glutamic acid was added and dispersed, followed by adjusting the pH of the solution to 9.4 by adding 20 % aqueous sodium hydroxide to dissolve glutamic acid. While being heated at about 65°C and agitated, the aqueous solution was gradually dropped with 58 g of glycidol over 30 minutes. After the dropping finished, the solution was still left at 65°C under agitation for 6 hours to complete reaction. After completion of the reaction, the reaction solution was cooled down to a room temperature and then its pH was adjusted to 6.6 by adding hydrochloric acid, followed by concentration under a reduced pressure to obtain 241g of glyceryl derivative of glutamic acid. 19% of the product can be considered as sodium chloride yielded by the neutralization.

Contents of total nitrogen, total carbon and amino nitrogen were measured on the glyceryl derivative of glutamic acid and glutamic acid employed as raw material. The results were as follows. Since glutamic acid is hardly soluble in water, sodium glutamate 1 hydrate was used for analysis instead.

| Glyceryl derivative of glutamic acid | |
|---|---|
| Total nitrogen content | 4.57 % |
| Total carbon content | 31.32 % |
| Amino nitrogen content | 1.21 % |

| Sodium glutamate 1 hydrate(The enclosed by parenthesis is a calculated value) | |
|---|---|
| Total nitrogen content | 7.36 % (7.49 %) |
| Total carbon content | 31.25 % (32.09 %) |
| Amino nitrogen content | 7.36 % (7.49 %) |

According to the above results, it was confirmed that 70 % or more of the amino groups in the glutamic acid were reacted with glyceryl group, and it was understood that the major component of the obtained glyceryl derivative of glutamic acid was sodium N-glycerylglutamate.

Referential Example 1 and Comparative Referential Example 1

The moisture-absorption ability and moisturizing effect of amino acid N-glyceryl derivatives produced in Synthesis Examples of 1 to 10, 13 and 14, were evaluated by moisture-absorption ability measurement or moisturizing effect measurement, respectively described below. As Comparative Referential Example 1, a moisture-absorption ability and moisturizing effect were also measured on polyols or amino acids which are frequently applied for moisturizer of cosmetics.

[Method of Moisture-absorption ability measurement carried out by employing keratin powder (ground wool)]

① Mixing 2.0 g of keratin powder with 5.0 g of water;
② putting 1.0 g of the mixture in a vessel;
③ dispersing each sample so that sample concentration (excluding water) being 0.3 g;
④ evaporating water in a drier until weight of the sample becoming unchanged; and then
⑤ holding the sample in a constant-humidity bath at 20°C under 79.2 % of relative humidity to measure moisture-absorption ability from the change of sample weight after 48 hours elapsed. For the constant-humidity bath of 79.2 % of relative humidity, saturated aqueous ammonium chloride solution was employed.

Moisture-absorption ability was evaluated by the following criteria.

Moisture-absorption ability rating criteria

◎ : Amount of absorbed water per 1 g of keratin powder is 0.60 g or more.
○: Amount of absorbed water per 1 g of keratin powder is 0.50 g or more and less than 0.60 g
Δ : Amount of absorbed water per 1 g of keratin powder is 0.40 g or more and less than 0.50 g
× : Amount of absorbed water per 1 g of keratin powder is less than 0.40 g

[Method of moisturizing effect measurement carried out by employing keratin powder (ground wool)]

Moisturizing effect measurement was carried out in a following manner that the sample employed in the above moisture-absorption ability measurement (i.e. the sample having been held in a constant-humidity bath used in the above step ⑤ under 79.2 % of relative humidity for 48 hours) was further held in a constant-humidity bath at 20°C under 20.0 % of relative humidity to measure moisturizing effect according to the change of sample weight after 6 hours elapsed. For the constant-humidity bath under 20.0 % of relative humidity, saturated aqueous potassium acetate solution was employed.

$$\text{Moisturizing effect} = (B/A) \times 100$$

, wherein:

A : Amount of absorbed water per 1 g of keratin powder when being left under 79.2 % of relative humidity for 48 hours;

B : Amount of absorbed water per 1 g of keratin powder when being left under 79.2 % of relative humidity for 48 hours and then left under 20.0 % of relative humidity for 6 hours

Moisturizing effect was evaluated by the following criteria.

Moisturizing effect rating criteria

◎ : 60 or more
○: 50 or more and less than 60
Δ : 40 or more and less than 50
× : Less than 40

The results of the measurements of moisture-absorption ability and moisturizing effect of amino acid N-glyceryl derivatives, according to the above measurement methods are shown in Table 1, and the results of the measurements of moisture-absorption ability and moisturizing effect of polyols or amino acids, as Comparative Referential Examples, are shown in Table 2.

Referential Example 2 and Comparative Referential Example 2

The moisturizing effect on skin of amino acid N-glyceryl derivatives produced in Synthesis Examples of 1 to 10, 13 and 14, was evaluated by ten male and female panelists. Evaluation was carried out in a manner that forearm of panelists was washed and then applied with 100 μl of aqueous amino acid N-glyceryl derivative solution prepared in 5 % concentration. Then, conductance value of the skin was measured by SKICON200-EX (manufactured by IBS K.K.) before the application and in 20 minutes elapsed after the application. As Comparative Referential Example, a moisturizing effect on skin was also measured on polyols or amino acids which are frequently applied for cosmetics.
Moisturizing effect on skin was evaluated by the following criteria.

Moisturizing effect on skin rating criteria

○: Conductance changing ratio is 10 % more
□: Conductance changing ratio is 3 % or more and less than 10 %
× : Conductance changing ratio is less than 3 %

The results of the measurements of moisturizing effect on skin of amino acid N-glyceryl derivatives, according to the above measurement methods are shown in Table 1, and the results of the measurements of moisturizing effect on skin of polyols or amino acids, as Comparative Referential Examples, are shown in Table 2.

Referential Example 3 and Comparative Referential Example 3

The stability of amino acid N-glyceryl derivatives produced in Synthesis Examples of 1 to 10, 13 and 14, was evaluated by the stability evaluation method described below. As Comparative Referential Example, stability of polyols or amino acids, which are frequently applied for cosmetics as moisturizers, was also measured.

[Method of stability evaluation]

In 50 ml of screw tube, 15 g of 5 % by weight sample solution was put in and covered by airtight sealing, followed by being held in a constant-temperature bath at 50°C for 1 month. After 1 month being elapsed, the sealing was opened, followed by odor of samples being evaluated by 5 panelists according to the following criteria. Although, among the amino acid samples for comparison; leucine and phenylalanine did not form homogeneous solution, the solutions thereof were subjected to evaluation as themselves.

Odor rating criteria

3 : No degradation odor
2 : Almost no degradation odor
1 : Strong degradation odor

Odor evaluation value of each sample was the average of values determined by 5 panelists; the evaluation result was represented by the following marks rated according to respective criteria.

○: 2.4 or more and less than 3
□: 1.8 or more and less than 2.4
×: Less than 1.8

The results of the measurements of stability of amino acid N-glyceryl derivatives, according to the above measurement methods are shown in Table 1, and the results of the measurements of stability of polyols or amino acids, as Comparative Referential Examples, are shown in Table 2.

Table 1

| Amino acid glyceryl derivative (Major component (Synthesis Example No.) | Referential Example 1 | | Referential Example 2 | Referential Example 3 |
|---|---|---|---|---|
| | Moisture-absorption ability | Moisturizing effect | Moisturizing effect on skin | Stability |
| Glyceryl derivative of glycine (Synthesis Example 1) | ◎ | ◎ | ○ | Δ |
| Glyceryl derivative of alanine (Synthesis Example 2) | ◎ | ○ | ○ | Δ |
| Glyceryl derivative of sarcosine (Synthesis Example 3) | ◎ | ◎ | ○ | ○ |
| Glyceryl derivative of phenylalanine (Synthesis Example 4) | ◎ | ○ | ○ | ○ |
| Glyceryl derivative of leucine (Synthesis Example 5) | ◎ | ○ | ○ | ○ |
| Glyceryl derivative of proline (Synthesis Example 6) | ◎ | ◎ | ○ | Δ |

Table 1 (continued)

| Amino acid glyceryl derivative (Major component (Synthesis Example No.) | Referential Example 1 | | Referential Example 2 | Referential Example 3 |
|---|---|---|---|---|
| | Moisture-absorption ability | Moisturizing effect | Moisturizing effect on skin | Stability |
| Glyceryl derivative of serine (Synthesis Example 7) | ◎ | ◎ | ○ | ○ |
| Glyceryl derivative of lysine (Synthesis Example 8) | ◎ | ◎ | ◎ | Δ |
| Glyceryl derivative of arginine (Synthesis Example 9) | ◎ | ◎ | ◎ | ◎ |
| Glyceryl derivative of histidine (Synthesis Example 10) | ◎ | ◎ | ◎ | ◎ |
| Glyceryl derivative of aspartic acid (Synthesis Example 13) | ◎ | ◎ | ◎ | ○ |
| Glyceryl derivative of glutamic acid (Synthesis Example 14) | ◎ | ◎ | ◎ | ○ |

Table 2

| Sample for comparison | Comparative referential example 1 | | Comparatve referential example 1 | Comparative referential example 1 |
|---|---|---|---|---|
| | Moisture-absorption ability | Moisturizing effect | Moisturizing effect on skin | Stability |
| 1,3-Butylene glycol | Δ | Δ | Δ | Δ |
| Glycerin | ○ | ○ | ○ | ○ |
| Dipropyleneglycol | Δ | Δ | Δ | × |
| Glycine | Δ | Δ | Δ | Δ |
| Sarcosine | Δ | Δ | × | Δ |
| Phenylalanine | × | × | × | Δ |
| Proline | ○ | ○ | Δ | × |
| Serine | Δ | Δ | × | Δ |
| Leucine | - | - | × | Δ |

Table 2 (continued)

| Sample for comparison | Comparative referential example 1 | | Comparatve referential example 1 | Comparative referential example 1 |
|---|---|---|---|---|
| | Moisture-absorption ability | Moisturizing effect | Moisturizing effect on skin | Stability |
| Alanine | - | - | - | × |
| Lysine hydrochloride | Δ | Δ | Δ | Δ |
| Arginine hydrochloride | ○ | ○ | ○ | ○ |
| Histidine hdrochloride | Δ | Δ | Δ | Δ |
| Polyethylene glycol 400 | - | - | - | × |
| Sodium aspartate hydrate | Δ | Δ | Δ | Δ |
| Sodium glutamate hydrate | Δ | Δ | ○ | Δ |

From the results in Table 1 and Table 2, it is apparent that the amino acid N-glyceryl derivatives produced in any one of Synthesis Examples of 1 to 10, 13 and 14 is superior in moisture-absorption ability, moisturizing effect, and moisturizing effect on skin to polyols or amino acids evaluated in Comparative Referential Examples, and same as or superior to the polyols or amino acids in stability.

Example 1 to 3 and Comparative Example 1 to 3

Cosmetic lotions exhibited in Table 3 were prepared. Then the feel of stickiness and moistness on each cosmetic lotion were evaluated by 10 panelists.

Table 3

| | Example 1 | Comparative Example 1 | Example 2 | Comparative Example 2 | Example 3 | Comparative Example 3 |
|---|---|---|---|---|---|---|
| Glyceryl derivative of glycine of Synthesis Example 1 | 2.0 | - | - | - | - | - |
| Glyceryl derivative of lysine of Synthesis Example 8 | - | - | 1.0 | - | - | - |
| Glyceryl derivative of aspartic acid of Synthesis Example 13 | - | - | - | - | 2.0 | - |
| Glycerin | - | 2.0 | - | 1.0 | - | 2.0 |

Table 3 (continued)

| | Example 1 | Comparative Example 1 | Example 2 | Comparative Example 2 | Example 3 | Comparative Example 3 |
|---|---|---|---|---|---|---|
| Oleyl alcohol | 0.1 | 0.1 | 0.1 | 0.1 | 0.06 | 0.06 |
| Polyoxyethylene (20) sorbitan laurate | 0.3 | 0.3 | 0.3 | 0.3 | 0.3 | 0.3 |
| Polyoxyethylene (15) Lauryl ether | 0.5 | 0.5 | 0.5 | 0.5 | 0.5 | 0.5 |
| Ethanol | 5.0 | 5.0 | 5.0 | 5.0 | 5.0 | 5.0 |
| Perfume | Optiona amount | Optional amount | Optiona amount | Optional amount | Optional amount | Optional amount |
| Antiseptic | Optiona amount | Optional amount | Optiona amount | Optional amount | Optiona amount | Optional amount |
| Purified water | Amount adjuste to 100 in total | Amount adjusted to 100 in total | Amount adjuste to 100 in total | Amount adjusted to 100 in total | Amount adjusted to 100 in total | Amount adjusted to 100 in total |

Evaluation was carried out in a manner that forearm of 10 panelists was applied with 1 ml by each of respective cosmetic lotions, followed by evaluation on the feels of stickiness and moistness according to the following evaluation criteria.

Stickiness feel rating criteria

3 : Not sticky
2 : Not so sticky
1 : Sticky

Moistness feel rating criteria

3 : Moist
2 : Somewhat moist
1 : Not moist

The evaluation results are exhibited in Table 4 in the average value of results obtained by 10 panelists.

Table 4

| | Example 1 | Comparative Example 1 | Example 2 | Comparative Example 2 | Example 3 | Comparative Example 2 |
|---|---|---|---|---|---|---|
| Sticky feel | 2.6 | 1.3 | 2.5 | 1.2 | 2.4 | 1.4 |
| Moist feel | 2.7 | 2.2 | 2.6 | 2.1 | 2.5 | 1.8 |

As apparent from the results in Table 4, the evaluation values of cosmetic lotions of Example 1, 2 and 3 are

superior in both feels of stickiness and moistness to that of cosmetic lotions of Comparative Example 1, 2 and 3.

Example 4 to 6 and Comparative Example 4 to 6

Milky lotions exhibited in Table 5 were prepared. Then, on applying each milky lotion on skin, the feels of stickiness and moistness were evaluated by 10 panelists.

Table 5

| | Example 4 | Comparative Example 4 | Example 5 | Comparative Example 5 | Example 6 | Comparative Example 6 |
|---|---|---|---|---|---|---|
| Glyceryl derivative of sarcosine of Synthesis Example 3 | 5.0 | - | - | - | - | - |
| Glyceryl derivative of arginine of Synthesis Example 9 | - | - | 1.0 | - | - | - |
| Glyceryl derivative of glutamic acid of Synthesis Example 14 | - | - | - | - | 5.0 | - |
| Dipropylene glycol | - | 5.0 | - | 1.0 | - | 5.0 |
| Micro crystalline wax | 1.0 | 1.0 | 1.0 | 1.0 | 1.0 | 1.0 |
| Beewax | 2.0 | 2.0 | 2.0 | 2.0 | 2.0 | 2.0 |
| lanolin | 2.0 | 2.0 | 2.0 | 2.0 | 1.0 | 1.0 |
| liquid paraffin | 18.0 | 18.0 | 18.0 | 18.0 | 6.0 | 6.0 |
| squalane | 12.0 | 12.0 | 12.0 | 12.0 | 8.0 | 8.0 |
| Sorbitan sesquioleate | 4.0 | 4.0 | 4.0 | 4.0 | 4.0 | 4.0 |
| Polyoxyethylene (20) sorbitan monooleate | 1.0 | 1.0 | 1.0 | 1.0 | 1.0 | 1.0 |
| Perfume | Optional amount | Optional amount | Optional amount | Optional amount | Optional amount | Optional amount |
| Antiseptic | Optional amount | Optional amount | Optional amount | Optional amount | Optional amount | Optional amount |

Table 5 (continued)

| | Example 4 | Comparative Example 4 | Example 5 | Comparative Example 5 | Example 6 | Comparative Example 6 |
|---|---|---|---|---|---|---|
| Antioxidant | Optional amount | Optional amount | Optional amount | Optional amount | Optional amount | Optional amount |
| Purified water | Amount adjusted to 100 | Amount adjusted to 100 | Amount adjusted to 100 | Amount adjusted to 100 | Amount adjusted to 100 | Amount adjusted to 100 |

Evaluation was carried out in a manner that forearm of 10 panelists was applied with 1 g by each of respective milky lotions, followed by evaluation on the feels o stickiness and moistness according to the same evaluation criteria as those in Examples 1-3. The evaluation results are exhibited in Table 7 in the average value of results obtained by 10 panelists.

Table 6

| | Example 4 | Comparative Example 4 | Example 5 | Comparative Example 5 | Example 6 | Comparative Example 6 |
|---|---|---|---|---|---|---|
| Sticky feel | 2.7 | 1.4 | 2.5 | 1.2 | 2.4 | 1.3 |
| Moist feel | 2.6 | 1.8 | 2.4 | 1.6 | 2.6 | 1.4 |

As apparent from the results in Table 6, the evaluation values of milky lotions of Example 4, 5 and 6 are superior in both feels of stickiness and moistness to that of milky lotions of Comparative Example 4, 5 and 6.

Example 7 to 9 and Comparative Example 7 to 9

Creams exhibited in Table 7 were prepared. Then, on applying each cream on skin, the feels of stickiness and moistness were evaluated by 10 panelists.

Table 7

| | Example 7 | Comparative Example 7 | Example 8 | Comparative Example 8 | Example 9 | Comparative Example 9 |
|---|---|---|---|---|---|---|
| Glyceryl derivative of leucine of Synthesis Example 5 | 15.0 | - | - | - | - | - |
| Leucine | - | 5.0 | - | - | - | - |
| Glyceryl derivative of histidine of Synthesis Example 10 | - | - | 6.0 | - | - | - |
| Histidine hydrochloride (1 1 hydrate) | - | - | - | 2.0 | - | - |

Table 7 (continued)

| | Example 7 | Comparative Example 7 | Example 8 | Comparative Example 8 | Example 9 | Comparative Example 9 |
|---|---|---|---|---|---|---|
| Glyceryl derivative of aspartic acid of Synthesis Example 13 | - | - | - | - | 6.0 | - |
| Sodium aspartate (1 hydrate) | - | - | - | - | - | 2.0 |
| Glycerin | - | 10.0 | - | 4.0 | - | 4.0 |
| Stearyl alcohol | 7.0 | 7.0 | 7.0 | 7.0 | 6.0 | 6.0 |
| Stearic acid | 2.0 | 2.0 | 2.0 | 2.0 | 2.0 | 2.0 |
| lanolin | 2.0 | 2.0 | 2.0 | 2.0 | 2.0 | 2.0 |
| squalane | 5.0 | 5. 0 | 5. 0 | 5. 0 | 4. 0 | 4.0 |
| 2-octyldodecyl alcohol | 6.0 | 6.0 | 6.0 | 6.0 | 6.0 | 6.0 |
| Polyoxyethylene (25) cetylether | 3.0 | 3.0 | 3.0 | 3.0 | 3.0 | 3.0 |
| Glyceryl monostearate | 2. 0 | 2.0 | 2.0 | 2.0 | 2.0 | 2.0 |
| Ascorbyl glucoside | 2.0 | 2.0 | 2.0 | 2.0 | 2.0 | 2.0 |
| Perfume | Optional amount | Optional amount | Optional amount | Optional amount | Optional amount | Optional amount |
| Antiseptic | Optional amount | Optional amount | Optional amount | Optional amount | Optional amount | Optional amount |
| Purified water | Amount adjusted to 100 | Amount adjusted to 100 | Amount adjusted to 100 | Amount adjusted to 100 | Amount adjusted to 100 | Amount adjusted to 100 |

Evaluation was carried out in a manner that forearm of 10 panelists was applied with 1 g by each of respective creams, followed by evaluation on the feels of stickiness and moistness according to the same evaluation criteria as those in Examples 1-3. The evaluation results are exhibited in Table 8 in the average value of results obtained by 10 panelists.

Table 8

| | Example 7 | Comparative Example 7 | Example 8 | Comparative Example 8 | Example 9 | Comparative Example 9 |
|---|---|---|---|---|---|---|
| Sticky feel | 2.5 | 1.5 | 2.6 | 1.6 | 2.6 | 1.6 |
| Moist feel | 2.4 | 1.7 | 2.5 | 1.8 | 2.5 | 1.8 |

As apparent from the results in Table 8, the evaluation values of creams of Example 7, 8 and 9 are superior in both feels of stickiness and moistness to that of milky lotions of Comparative Example 7, 8 and 9.

Examples 10-12 and Comparative Examples 10-12

Hair creams exhibited in Table 9 were prepared. Then, on applying each hair cream on hair washed with shampoo, the gloss, feels of moistness and combability of hair were evaluated.

Table 9

| | Example 10 | Comparative Example 10 | Example 11 | Comparative Example 11 | Example 12 | Comparative Example 12 |
|---|---|---|---|---|---|---|
| Glyceryl derivative of phenylalanine of Synthesis Example 4 | 5.0 | - | - | - | - | - |
| Phenylalanine | - | 2.0 | - | - | - | - |
| Glyceryl derivative of lysine of Synthesis Example 8 | - | - | 8.0 | - | - | - |
| Glyceryl derivative of arginine of Synthesis Example 9 | - | - | 4.0 | - | - | - |
| Lysine hydrochloride | - | - | - | 4.0 | - | - |
| Arginine hydrochloride | - | - | - | 2.0 | - | - |
| Glyceryl derivative of glutamic acid of Synthesis Example 14 | - | - | - | - | 4.0 | - |
| Sodium Glutamate (1 hydrate) | - | - | - | - | - | 2.0 |

Table 9 (continued)

| | Example 10 | Comparative Example 10 | Example 11 | Comparative Example 11 | Example 12 | Comparative Example 12 |
|---|---|---|---|---|---|---|
| Glycerin | - | 3.0 | - | 6.0 | - | 2.0 |
| Liquid paraffin | 15.0 | 15.0 | 15.0 | 15.0 | 16.0 | 16.0 |
| Vaseline | 15.0 | 15.0 | 15.0 | 15.0 | 16.0 | 16.0 |
| Carboxyl vinyl polymer | 0.1 | 0.1 | 0.1 | 0.1 | 0.1 | 0.1 |
| Xanthan Gum | 0.1 | 0.1 | 0.1 | 0.1 | 0.1 | 0.1 |
| Polyoxyethylene (40) hydrogenated castor oil | 3.0 | 3.0 | 3.0 | 3.0 | 3.0 | 3.0 |
| Sodium hydroxide | 0.05 | 0.05 | 0.05 | 0.05 | 0.05 | 0.05 |
| Perfume | Optional amount | Optional amount | Optional amount | Optional amount | Optional amount | Optional amount |
| Antiseptic | Optional amount | Optional amount | Optional amount | Optional amount | Optional amount | Optional amount |
| Purified water | Amount adjusted to 100 | Amount adjusted to 100 | Amount adjusted to 100 | Amount adjusted to 100 | Amount adjusted to 100 | Amount adjusted to 100 |

Before the treatment by hair cream of the above Examples 10-12 or Comparative examples 10-12, two bundles of hair, each bundle being the length of 15 cm and the weight of 1 g; were prepared, followed by washing each bundle with 2% aqueous solution of sodium polyoxyethylene (3) laurylether sulfate, rinsing it with hot stream water, and then being dried in air. On each rinsed hair bundle, 1g of hair cream for Example 10 or Comparative example 10, 0.5g of hair cream for Examples 11, 12 or Comparative examples 11, 12, was applied with spreading thoroughly, and then the bundle was dried with a hair drier. The dried hair was evaluated about gloss, moist feel and combability, etc. by 10 panelists according to the criteria mentioned below. The evaluation results are exhibited in Table 10 in the average value of results obtained by 10 panelists.

Rating criteria for treated hair

5 : Excellent
4 : Good
3 : Fair
2 : Bad
1 : Very bad

Table 10

| | Example 10 | Comparative Example 10 | Example 11 | Comparative Example 11 | Example 12 | Comparative Example 12 |
|---|---|---|---|---|---|---|
| gloss | 4.5 | 3.2 | 4.3 | 3.1 | 4.2 | 3.0 |
| moist feel | 4.6 | 3.4 | 4.5 | 3.2 | 4.4 | 3.3 |
| smoothness | - | - | 4.3 | 2.9 | - | - |
| combability | 4.2 | 2.7 | 4.1 | 2.8 | 4.0 | 2.9 |

As apparent from the results in Table 10, the evaluation values of hair applied with the hair creams of Examples 10-12 are superior in any of gloss of hair, moist feel and combability to hair applied with the hair creams of Comparative Examples 10-12.

Examples 13-15 and Comparative Examples 13-15

Hair conditioners exhibited in Table 11 were prepared. Then, on applying each hair conditioners on hair rinsed with shampoo, the gloss, moist feel and combability of hair were evaluated.

Table 11

| | Example 13 | Comparative Example 13 |
|---|---|---|
| Glyceryl derivative of leucine of Synthesis Example 5 | 3.0 | - |
| Propylene glycol | - | 3.0 |
| Stearyl trimethylammonium chloride | 1.0 | 1.0 |
| Cetanol | 1.5 | 1.5 |
| Stearyl alcohol | 1.0 | 1.0 |
| Methyl polysiloxane | 2.0 | 2.0 |
| Liquid paraffin | 1.0 | 1.0 |
| Perfume | Optional amount | Optional amount |
| Colorant | Optional amount | Optional amount |
| Antiseptic | Optional amount | Optional amount |
| Purified water | Amount adjusted to 100 | Amount adjusted to 100 |

Before the treatment by hair conditioners of the above Example 13 or Comparative example 13, two bundles of hair, each bundle being the length of 15 cm and the weight of 1 g; were prepared, followed by washing each bundle with 2% aqueous solution of sodium polyoxyethylene (3) laurylether sulfate, rinsing it with hot stream water, and then being dried in air. On each washed hair bundle, 2g of hair conditioner of Example 13 or Comparative example 13 was applied with spreading thoroughly, and then the bundle was dried with a hair drier. The dried hair was evaluated about gloss, moist feel and combability by 10 panelists according to the same criteria as in Examples 10-12. The evaluation results are exhibited in Table 12 in the average value of results obtained by 10 panelists.

Table 12

| | Example 13 | Comparative Example 13 |
|---|---|---|
| gloss | 4.4 | 3.1 |
| moist feel | 4.5 | 2.9 |
| combability | 4.3 | 2.7 |

As apparent from the results in Table 12, the evaluation values of hair applied with the hair conditioner of Example 13 are superior in any of gloss of hair, moist feel and combability to hair applied with the hair conditioner of Comparative Example 13.

Example 14 and Comparative Example 14

Hair rinse having a composition exhibited in Table 13 can be prepared. When each of hair rinses is applied on hair washed with shampoo, and evaluated gloss of hair, moist feel and combability to hair according to the evaluation method mentioned below, it can be expected that hair applied with the hair conditioner of Example 14 are superior in any of gloss of hair, moist feel and combability to hair applied with the hair conditioner of Comparative Example 14.

Evaluation method

Before the treatment by hair rinses of the above Example 14 or Comparative example 14, two bundles of hair, each bundle being the length of 15 cm and the weight of 1 g; were prepared, followed by washing each bundle with 2% aqueous solution of so dium polyoxyethylene(3)laurylether sulfate, rinsing it with ho t stream water, and then being dried in air. On each washed ha ir bundle, 0.5g of hair rinse of Example 14 or Comparative exam ple 14 was applied with spreading thoroughly, and then the bun dle was dried with a hair drier. The dried hair was evaluated about gloss, moist feel and combability by 10 panelists accord ing to the same criteria as in Examples 10-12.

Table 13

| | Example14 | Comparative Example 14 |
|---|---|---|
| Glyceryl derivative of arginine of Synthesis Example 11 | 3.0 | - |
| Propylene glycol | - | 3.0 |
| Stearyl trimethylammonium chloride | 1.0 | 1.0 1.5 |
| Cetanol | 1.5 | |
| Stearyl alcohol | 1.0 | 1.0 |
| Dimethyl polysiloxane | 2.0 | 2.0 |
| Liquid paraffin | 1.0 | 1.0 |
| Perfume | Optional amount | Optional amount |
| Colorant | Optional amount | Optional amount |
| Antiseptic | Optional amount | Optional amount |
| Purified water | Amount adjusted to 100 | Amount adjusted to 100 |

Example 15-17 and Comparative Example 15-17

Shampoo having a composition exhibited in Table 14 can be prepared. When each of shampoo is applied on hair, and evaluated gloss of hair, moist feel and combability to hair according to the evaluation method mentioned below, it can be expected that hair applied with the shampoo of any of Examples 15-17 are superior in any of gloss of hair, moist feel and combability to hair applied with the hair conditioner of any of Comparative Example 15-17.

Evaluation method

Two bundles of hair, each bundle being the length of 15 cm and the weight of 1 g; were prepared. Each bundle was washed for 1 minute using the shampoo, and rinsed with hot stream water. After repeating the washing with shampoo and rinse treatment five times, gloss, moist feel and combability were evaluated by 10 panelists according to the same criteria as in Examples 10-12.

Table 14

| | Example 15 | Comparative Example 15 | Example 16 | Comparative Example 16 | Example 17 | Comparative Example 17 |
|---|---|---|---|---|---|---|
| Glyceryl derivative of Sarcosine of Synthesis Example 3 | 3.0 | - | - | - | - | - |
| Sarcosine | - | 3.0 | - | - | - | - |
| Glyceryl derivative of tryptophan of Synthesis Example 12 | - | - | 4.0 | - | - | - |
| Glyceryl derivative of glutamic acid of Synthesis Example 14 | - | - | - | - | 0.1 | - |
| Glycerin | - | - | - | 4.0 | - | 0.1 |
| Triethanol amine poly oxyethlene (3) lauryl ether sulfate (30%) | 10.0 | 10.0 | 10.0 | 10.0 | 9.0 | 9.0 |
| Sodium polyoxyethlene (3) laurylether sulfate (30%) | 25.0 | 25.0 | 25.0 | 25.0 | 22.0 | 22.0 |
| Coconut fatty acid diethanol amide | 5.0 | 5.0 | 5.0 | 5.0 | 5.0 | 5.0 |
| Lauryl dimethyl amino acetic acid (35%) | 7.0 | 7.0 | 7.0 | 7.0 | 7.0 | 7.0 |
| Cationic cellulose | 0.1 | 0.1 | 0.1 | 0.1 | 0.1 | 0.1 |
| Ethyleneglycol distearate | 2.0 | 2.0 | 2.0 | 2.0 | 2.0 | 2.0 |
| Perfume | Optional amount | Optional amount | Optional amount | Optional amount | Optional amount | Optional amount |

Table 14 (continued)

| | Example 15 | Comparative Example 15 | Example 16 | Comparative Example 16 | Example 17 | Comparative Example 17 |
|---|---|---|---|---|---|---|
| Antiseptic | Optional amount | Optional amount | Optional amount | Optional amount | Optional amount | Optional amount |
| Purified water | Amount adjusted to 100 | Amount adjusted to 100 | Amount adjusted to 100 | Amount adjusted to 100 | Amount adjusted to 100 | Amount adjusted to 100 |

Examples 18-20 and Comparative Examples 18-20

No. 1 waving agent for permanent wave having a composition exhibited in Table 15 were prepared. Each of the No.1 waving agent for permanent wave and No.2 waving agent for permanent wave comprising 6% aqueous sodium bromate solution were used for permanent wave treatment, and the gloss, moist feel and combability of hair after the permanent wave treatment were evaluated.

Table 15

| | Example 18 | Comparative Example 18 | Example 19 | Comparative Example 19 | Example 20 | Comparative Example 20 |
|---|---|---|---|---|---|---|
| Glyceryl derivative of proline of Synthesis Example 6 | 6.0 | - | - | - | - | - |
| Proline | - | 3.5 | - | - | - | - |
| Glyceryl derivative of histidine of Synthesis Example 10 | - | - | 2.0 | - | - | - |
| Glyceryl derivative of aspartic acid of Synthesis Example 13 | - | - | - | - | 2.0 | - |
| Glycerin | - | 2.5 | - | 2.0 | - | 2.0 |
| Ammonium thioglycolate | 12.0 | 12.0 | 12.0 | 12.0 | 12.0 | 12.0 |
| Cetyl trimethyl ammonium chloride (30%) | 1.0 | 1.0 | 1.0 | 1.0 | 1.0 | 1.0 |
| Monoethnol amine | 1.8 | 1.8 | 1.8 | 1.8 | 1.8 | 1.8 |
| Polyoxyethlene (15) laurylether | 0.5 | 0.5 | 0.5 | 0.5 | 0.5 | 0.5 |

Table 15 (continued)

| | Example 18 | Comparative Example 18 | Example 19 | Comparative Example 19 | Example 20 | Comparative Example 20 |
|---|---|---|---|---|---|---|
| EDTA-2Na | 0.1 | 0.1 | 0.1 | 0.1 | 0.1 | 0.1 |
| Aqueous ammonia(25%) | 1.6 | 1.6 | 1.6 | 1.6 | 1.6 | 1.6 |
| Purified water | Amount adjusted to 100 | Amount adjusted to 100 | Amount adjusted to 100 | Amount adjusted to 100 | Amount adjusted to 100 | Amount adjusted to 100 |

The above hair treatment with No.1 waving agent for permanent wave was conducted as follows. That is, hairs having the same length of 18 cm were rinsed with 2% aqueous solution of sodium polyoxyethylene(3)laurylether sulfate, and, then, rinsed with running tap water, followed by being dried in air.

Two bundles each consisting of 40 hairs were prepared from the hairs obtained above, and each of them was wound on a rod having length of 10cm and diameter of 1cm. On the hair bundle wound on the rod, 2ml of any of the No.1 waving agent for permanent wave of Examples 18-20 and Comparative examples 18-20 was applied, and, then, the bundle was wrapped with plastic film. Thereafter, the bundle was left as it was for 15 minutes, followed by rinsed gently with running tap water for 10 seconds.

Then, 2ml of No.2 waving agent for permanent wave was applied,and, the bundle was wrapped with plastic film. Thereafter ,the bundle was left as it for 15 minutes, followed by rinsing gently with running tap water.

Each rod was dried in a hot air drier. After dried, the hair bundle was removed from the rod, and the dried hair was evaluated about gloss of hair, moist feel, springness and combability by 10 panelists according to the same criteria as in Examples 10-12. The evaluation results are exhibited in Table 16 in the average value of results obtained by 10 panelists.

Table 16

| | Example 18 | Comparative Example 18 | Example 19 | Comparative Example 19 | Example 20 | Comparative Example 20 |
|---|---|---|---|---|---|---|
| gloss | 4.3 | 3.0 | 4.1 | 2.8 | 4.0 | 2.9 |
| moist feel | 4.2 | 3.2 | 4.0 | 3.0 | 4.1 | 2.9 |
| Spriginess | 4.0 | 2.9 | - | - | - | - |
| smoothness | 4.1 | 3.3 | 3.8 | 2.7 | 3.9 | 2.8 |
| combability | - | - | 3.9 | 3.1 | 3.8 | 3.0 |

As apparent from the results in Table 16, the evaluation values of hair applied with the No. 1 waving agent for permanent of Examples 18-20 are same as or superior to hair applied with the No.1 waving agent for permanent of Comparative Examples 18-20 in any of gloss of hair, moist feel and combability.

Examples 21, 22 and Comparative Examples 21, 22

Two of hair colorlants of oxidation type hair dye having a composition exhibited in Table 17 were prepared. Each of the hair colorlants of oxidation type hair dye and each of color developers of oxidation type hair dye exhibited in Table 18 below were mixed and applied for hair dyeing, and then, the gloss, moist feel and combability of hair were evaluated.

Table 17

| | Example 21 | Comparative Example 21 | Example 22 | Comparative Example 22 |
|---|---|---|---|---|
| Glyceryl derivative of arginine of Synthesis Example 9 | 1.5 | - | - | - |
| Arginine hydrochloride | - | 1.5 | - | - |
| Glyceryl derivative of glutamic acid of Synthesis Example 14 | - | - | 1.5 | - |
| Sodium glutamate (1 hydrate) | - | - | - | 1.5 |
| p-phenylene diamine | 3.0 | 3.0 | 3.0 | 3.0 |
| Resorcinol | 0.5 | 0.5 | 0.5 | 0.5 |
| Oleic acid | 20.0 | 20.0 | 20.0 | 20.0 |
| Polyoxyethlene (10) oleylether | 15.0 | 15.0 | 15.0 | 15.0 |
| Isopropanol | 10.0 | 10.0 | 10.0 | 10.0 |
| Aqueous ammonia (28%) | 10.0 | 10.0 | 10.0 | 10.0 |
| Purified water | Amount adjusted to 100 | Amount adjusted to 100 | Amount adjusted to 100 | Amount adjusted to 100 |

Same color developers of oxidation type hair dye were us ed in Examples 21, 22 and Comparative examples 21, 22, and the compositions are as exhibited in Table 18.

Table 18

| Component | Composition |
|---|---|
| Stearic acid | 1.0 |
| Glycerin monostearate | 1.5 |
| Polyoxyethlene (10) oreylether | 1.0 |
| Aqueous hydroperoxide (3 5 %) | 17.0 |
| Purified water | Amount adjusted to 100 |

The above hair treatment with hair colorlants of oxidation type hair dye was conducted as follows. That is, two bundles of hair, each bundle being the length of 15 cm and the weight of 1 g; were prepared. Each bundle was washed with 2% aqueous solution of sodium polyoxyethylene(3)laurylether sulfate, and, then, rinsed with running tap water, followed by being dried in air. On each hair bundle obtained above, 2g of oxidation type hair dye, being prepared

by mixing each of the hair colorlants of oxidation type hair dye of Examples 21, 22 and Comparative examples 21, 22 with a color developer, was applied uniformly on each hair bundle. Then, the hair bundle was left as it was for 30 minutes, rinsed with hot water. Thereafter, the hair bundle was washed with 2% aqueous solution of sodium polyoxyethylene (3) laurylether sulfate, and, then, rinsed with running tap water, followed by being dried by hot air with a hair drier. The dried hair was evaluated about gloss of hair, moist feel and combability by 10 panelists according to the same criteria as in Examples 10-12. The evaluation results are exhibited in Table 19 in the average value of results obtained by 10 panelists.

Table 19

| | Example 21 | Comparative Example 21 | Example 22 | Comparative Example 22 |
|---|---|---|---|---|
| gloss | 3.8 | 2.8 | 3.7 | 2.7 |
| moist feel | 4.3 | 3.2 | 4.1 | 3.1 |
| combability | 3.9 | 2.9 | 3.8 | 2.8 |

As apparent from the results in Table 19, the evaluation values of hair treated with the oxidation type hair dye of any of Examples 21, 22 are same as or superior to hair treated with the oxidation type hair dye of any of Comparative Examples 21, 22 in any of gloss of hair, moist feel and combability.

Example 23

Using glyceryl derivative of arginine of Synthesis Example 9, a milky lotion exhibited in Table 20 can be prepared. When applied on skin, it is expected that the evaluation values of this milky lotion are superior in both feels of stickiness and moistness to those of milky lotion having the same compositions except for using glycerin in place of the glyceryl derivative of arginine of Synthesis Example 9.

Table 20

| | Example 23 |
|---|---|
| POE(20)POP(2) Cetylether | 0.8 |
| Alkylglucoside(*1) | 0.2 |
| Dimethyl polysiloxane(20mPa·s) | 2.0 |
| Macadamia nut oil | 1.0 |
| Liquid paraffin | 3.0 |
| Arbutin | 3.0 |
| Magnesium ascorbyl phosphate | 0.5 |
| Sodium hyaluronate | 0.01 |
| Trehalose | 0.5 |
| Xylitol | 0.5 |
| Glyceryl derivative of arginine of Synthesis Example 9 | 3.0 |
| Ethanol | 15.0 |
| Carboxyvinylpolymer | 0.3 |
| Hydroxypropylcellulose | 0.1 |
| 2-aminomethylpropanol | 0.1 |
| Antiseptic | Optional amount |
| Purified water | Amount adjusted to 100 |

*1; MONTANOV68 manufactured by Seppic Co., Ltd.

Example 24

Using glyceryl derivative of histidine of Synthesis Example 10, a cream exhibited in Table 21 can be prepared. When applied on skin, it is expected that the evaluation values of this cream are excellent in both feels of stickiness and moistness.

Table 21

| | Example 24 |
|---|---|
| POE(20) Oleylether | 1.5 |
| Glyceryl monostearate | 1.5 |
| Acrylic acid - alkyl methacrylate copolymer (2% aqueous solution)(*1) | 0.5 |
| Squalane | 15.0 |
| Jojoba oil | 3.0 |
| Ceramide | 0.01 |
| Cetostearyl alcohol | 3.0 |
| Ascorbyl dipalmitate | 0.1 |
| Sodium citrate | 0.03 |
| Xanthan Gum | 0.05 |
| Pullulan | 0.01 |
| Diglycerin | 0.01 |
| Glyceryl derivative of histidine of Synthesis Example 10 | 5.0 |
| Antiseptic | Optional amount |
| Perfume | Optional amount |
| Purified water | Amount adjusted to 100 |

*1 ; Nikko Chemicals Co., Ltd. PEMULEN TR-1

Example 25

Using glyceryl derivative of glycine of Synthesis Example 1, a sun screen cream exhibited in Table 22 can be prepared. When applied on skin, it is expected that the evaluation values of this sun screen cream are superior in both feels of stickiness and moistness to those of other sun screen creams having the same compositions except for using glycerin in place of the glyceryl derivative of glycine of Synthesis Example 1.

Table 22

| | Example 25 |
|---|---|
| Cetyldimethiconepolyol | 2.0 |
| Polyether modified silicone(*1) | 0.5 |
| Jojoba oil | 1.0 |
| Glyceryl tri-2-ethylhexanoate | 2.0 |
| Neopentylglycol dicaprate | 2.0 |
| Crosslinked methyl polysiloxane(*2) | 1.5 |
| Decamethylcyclopenta siloxane | 6.0 |
| UVB scattering agent paste(*3) | 20.0 |
| UVA scattering agent paste(*4) | 8.0 |
| 1,3-butylene glycol | 3.0 |
| Glyceryl derivative of glycine of Synthesis Example 1 | 3.0 |
| Sodium chloride | 1.0 |
| Antiseptic | Optional amount |
| Purified water | Amount adjusted to 100 |

*1 Toray Dow Corning Co., Ltd. BY22-012
*2 Shin-Etsu Chemical Co., Ltd. KSG-16
*3 IWASE COSFA Co., Ltd. Cosmeserve WP-UF
*4 IWASE COSFA Co., Ltd. Cosmeserve WPA-STD

Example 26

Using glyceryl derivative of aspartic acid of Synthesis Example 13, a body cream exhibited in Table 23 can be prepared. When applied on skin, it is expected that the evaluation values of this body cream are superior in both feels of stickiness and moistness to those of other body creams having the same compositions except for using glycerin in place of the glyceryl derivative of aspartic acid of Synthesis Example 13.

Table 23

| | Example26 |
|---|---|
| Acrylic acid - alkyl methacrylate copolymer (2% aqueous solution)(*1) | 10.0 |
| Hydrogenated soybean phospholipid | 1.0 |
| Polyethyleneglycol monostearate(40EO) | 1.0 |
| Sorbitan monostearate | 1.0 |
| Squalane | 8.0 |
| Octyldodecyl myristate | 4.0 |
| Cetanol | 2.0 |
| Methyl polysiloxane (350mPa·s) | 0.3 |
| Decamethylcyclopentapolysiloxane | 4.0 |
| Peony extract | 0.2 |
| Hydrolysed silk(*2) | 0.5 |
| Inulin stearate(*3) | 0.2 |
| Sepigel 305 manufactured by Seppic Co. , Ltd (*4) | 0.2 |
| Sodium hyaluronate | 0.01 |
| Glyceryl derivative of aspartic acid of Synthesis Example 13 | 5.0 |
| Perfume | Optional amount |
| Antiseptic | Optional amount |
| Purified water | Amount adjusted to 100 |

*1 Nikko Chemicals Co., Ltd. PEMULEN TR-1
*2 Seiwa Kasei Co., Ltd. Promois silk-100
*3 Chiba Seifun Co., Ltd. Rheopearl ISK
*4 Mixture of polyacrylamide, hydrogenated isobutene, Laureth-7 and water

## Claims

1. Cosmetics containing amino acid N-glyceryl derivative represented by the following formula (I) or salt thereof;

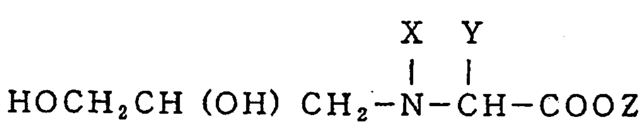

(I)

, wherein X represents hydrogen atom, $-CH_2CH(OH)CH_2OH$ group or alkyl group having 1 to 4 carbon atoms, Y represents a side chain of $\alpha$-amino acid, and Z represent hydrogen atom, alkali metal, ammonium, organic ammonium or $-CH_2CH(OH)CH_2OH$ group.

2. The cosmetics according to claim 1, wherein content of the amino acid N-glyceryl derivative represented by the formula (I) or salt thereof is 0.1 to 20 % by weight to total weight of the cosmetic.

3. The cosmetics according to claim 1 or claim 2, wherein the amino acid N-glyceryl derivative represented by the formula (I) or salt thereof is an glyceryl derivative of basic amino acid or salt thereof.

**4.** The cosmetics according to any one of claim 1 - 3, which are skin care cosmetics.

**5.** The cosmetics according to any one of claim 1 - 3, which are hair cosmetics.

## INTERNATIONAL SEARCH REPORT

International application No.
PCT/JP03/15520

**A. CLASSIFICATION OF SUBJECT MATTER**
Int.Cl[7] A61K7/00, 7/06, 7/075, 7/08, 7/09, 7/13

According to International Patent Classification (IPC) or to both national classification and IPC

**B. FIELDS SEARCHED**

Minimum documentation searched (classification system followed by classification symbols)
Int.Cl[7] A61K7/00-7/50

Documentation searched other than minimum documentation to the extent that such documents are included in the fields searched

Electronic data base consulted during the international search (name of data base and, where practicable, search terms used)
CAPLUS(STN), REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| A | JP 10-53570 A (Shiseido Co., Ltd.), 24 February, 1998 (24.02.98), Full text (Family: none) | 1-5 |
| A | JP 2001-181143 A (Lion Corp.), 03 July, 2001 (03.07.01), Full text (Family: none) | 1-5 |
| A | JP 2000-86454 A (Kose Corp.), 28 March, 2000 (28.03.00), Full text (Family: none) | 1-5 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 March, 2004 (10.03.04) | 23 March, 2004 (23.03.04) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/15520

C (Continuation). DOCUMENTS CONSIDERED TO BE RELEVANT

| Category* | Citation of document, with indication, where appropriate, of the relevant passages | Relevant to claim No. |
|---|---|---|
| A | JP 2000-86453 A (Kose Corp.),<br>28 March, 2000 (28.03.00),<br>Full text<br>(Family: none) | 1-5 |

Form PCT/ISA/210 (continuation of second sheet) (July 1998)